# EUROPÄISCHE PATENTANMELDUNG

(11) **EP 0 529 449 A1**
(43) Veröffentlichungstag der Anmeldung: **03.03.1993**
(21) Anmeldenummer: 92113955.6
(22) Anmeldetag: 17.08.1992
(51) Int. Cl.: C07D 277/80

(54) **Verfahren zur Herstellung von Bis-benzthiazolyl-alkyl-sulfenimiden**

(30) Priorität: 29.08.1991 DE 4128682
(71) Anmelder: BAYER AG, 51368 Leverkusen (DE)
(72) Erfinder: Sicheneder, Adolf, Dr., W-4047 Dormagen 5 (DE); Schlesmann, Harro, Dr., W-5068 Odenthal 3 (DE)

(57) **Zusammenfassung**

Bis-benzthiazolyl-alkyl-sulfenimide werden hergestellt, indem man Alkylamine mit gegebenenfalls substituierten 2-Mercaptobenzthiazolen in Gegenwart von Sauerstoff oder Sauerstoff-enthaltenden Gasen und Kupfer und/oder Kupferverbindungen in Gegenwart von inerten, organischen Lösungsmitteln umsetzt.

## Beschreibung

Die Erfindung betrifft ein Verfahren zur Herstellung von Bis-benzthiazolyl-alkyl-sulfenimiden aus gegebenenfalls substituierten 2-Mercaptobenzthiazolen und Alkylaminen durch katalysierte Sauerstoffoxidation. Die erhaltenen Bis-benzthiazolyl-alkyl-sulfenimide können als Vulkanisationsbeschleuniger eingesetzt werden.

Bis-benzthiazolyl-alkyl-sulfenimide sind bekannt und lassen sich durch Umsetzung von 2-Benzthiazolyl-alkylsulfenamide mit Säuren bzw. Säurederivaten, wie Säureanhydriden und Säurechloriden, herstellen. So wird beispielsweise in GB 12 88 701 und US 28 60 142 die Verwendung von Carbonsäureanhydriden bei der Herstellung der Bis-benzthiazolyl-alkyl-sulfenimiden beschrieben. Nach Ignatov et al. [Zh. Obshch. Khim. 47(5), 1096] sind Bis-benzthiazolyl-alkyl-sulfenimide auch durch Umsetzung der Sulfenamide mit Carbonsäurechloriden zugänglich. Nachteilig bei den obengenannten Verfahrensvarianten ist, daß äquivalente Mengen an Carbonsäureamiden entstehen. Die dabei entstehenden Carbonsäureamide müssen aus dem Reaktionsgemisch abgetrennt werden, wodurch sich die geschilderten Verfahrensvarianten wenig wirtschaftlich gestalten.

Weiterhin ist es möglich, durch Umsetzung von 2-Benzthiazolyl-sulfenylchlorid mit Alkylaminen (US 28 73 277) bzw. 2-Benzthiazolyl-alkyl-sulfenamiden (N.K. Sundholm, Ind. eng. Chem. 52, 239, 1960) Bis-benzthiazolyl-alkylsulfenimide herzustellen. Nachteilig bei diesem Verfahren ist die schlechte Zugänglichkeit der einzusetzenden Sulfenchloride, die in mäßigen Ausbeuten durch Umsetzung von entsprechenden Benzthiazolyldisulfiden mit Chlor erhalten werden.

Es wurde nun ein Verfahren zur Herstellung von Bis-benzthiazolyl-alkyl-sulfenimiden gefunden, das dadurch gekennzeichnet ist, daß man Alkylamine mit gegebenenfalls substituierten 2-Mercaptobenzthiazolen in Gegenwart von Sauerstoff oder Sauerstoff-enthaltenden Gasen und Kupfer und/oder Kupferverbindungen in Anwesenheit von organischen, inerten Lösungsmitteln umsetzt.

Entsprechend dem folgenden Reaktionsschema gelangt man ausgehend von Mercaptobenzthiazol und/oder den entsprechenden Derivaten von Mercaptobenzthiazol zu den gewünschten Verbindungen: 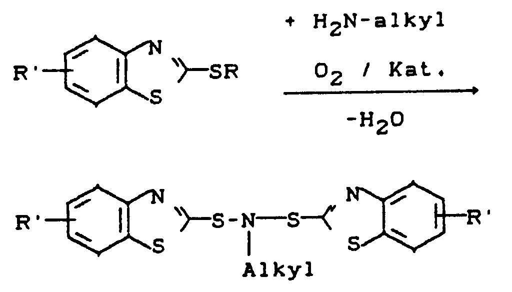
- R: = H, HN-Alkyl (C₄-C₁₂), 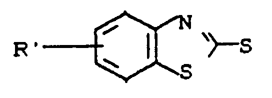
- R': = Alkyl (C₄-C₁₂), Hal (F, Cl, Br), NO₂, OR'' (R''= C₁-C₄-Alkyl)
Als inerte, organische Lösungsmittel können in das erfindungsgemäße Verfahren aprotische, organische Lösungsmittel eingesetzt werden. Beispielsweise können aliphatische und aromatische Kohlenwasserstoffe, wie Hexan, Cyclohexan, Benzol, Toluol, und/oder aliphatische Ether, wie Ethyl, Methyl- tert.-Butylether, und/oder aliphatische Nitrile, wie Acetonitril, und/oder Formamide, wie Dimethylformamid, in das erfindungsgemäße Verfahren eingesetzt werden. Darüber hinaus ist es möglich als Lösungsmittel aliphatische Amine, die am α-Kohlenstoffatom kein Proton tragen, in das erfindungsgemäße Verfahren einzusetzen. Als besonders vorteilhaft hat sich erwiesen tert.-Butylamin bzw. dessen Derivate als Lösungsmittel einzusetzen.

Die Lösungsmittel können einzeln oder im Gemisch untereinander eingesetzt werden.

Das erfindungsgemäße Verfahren wird in Gegenwart von Sauerstoff oder Sauerstoff-enthaltenden Gasen, wie Luft, durchgeführt. Dabei wird unter Druck von etwa 2 bis 100 bar, vorzugsweise 5 bis 30 bar, und bei einer Temperatur von ca. 10 bis 100°C, bevorzugt 40 bis 70°C, gearbeitet.

Als Alkylamine mit denen die Mercaptobenzthiazole umgesetzt werden, kommen alle aliphatischen Amine in Frage, die, wie zuvor erwähnt, am α-Kohlenstoffatom kein Proton tragen. Genannt werden z.B. tert.-Butylamin oder dessen Derivate, wie 2-Benzthiazolyl-tert.-butylsulfenamid, und tert.-Amylamin sowie dessen Derivate. Andere aliphatische Amine, die am α-Kohlenstoffatom kein Proton tragen, sind dem Fachmann bekannt und können ebenfalls eingesetzt werden. Neben 2-Mercaptobenzthiazol können die Benzthiazolyldisulfide und/oder 2-Benzthiazolylalkyl-sulfenamide eingesetzt werden.

Die Mercaptobenzthiazole sowie die Benzthiazolyldisulfide und die 2-Benzthiazolyl-alkyl-sulfenamide können noch, wie aus dem Formelschema ersichtlich, durch Alkylreste, Halogene, Nitrogruppen sowie Alkoxygruppen am Benzolring substituiert sein.

Das erfindungsgemäße Verfahren wird in Gegenwart von Kupfer und/oder Kupferverbindungen durchgeführt.

Als Kupferverbindungen kommen anorganische, organische, einfache oder komplexe Kupfersalze in Betracht, wie die Sulfate, Acetate und Acetylacetonate. Ganz besonders bevorzugt ist Kupfer-(I)-benzthiazolyl-2-mercaptid.

Die Kupferkatalysatoren können in Mengen von ca. 0,001 bis 1, bevorzugt 0,01 bis 1 Gew.-%, bezogen auf die eingesetzten Mercaptobenzthiazole, eingesetzt werden.

Die Menge an organischen, inerten Lösungsmitteln beträgt ca. 40 bis 90 Gew.-%, vorzugsweise 60 bis 80 Gew.-%, bezogen auf das Gesamtgewicht der Reaktionslösung.

Das erfindungsgemäße Verfahren kann sowohl kontinuierlich als auch diskontinuierlich in allen dafür geeigneten Reaktoren durchgeführt werdend Die optimalen Reaktionsbedingungen können leicht durch Vorversuche ermittelt werden.

### Beispiele

### Beispiel 1

In einem Stahlautoklaven wurden vorgelegt:

| | |
|---|---|
| 50 Gew.-Teile | Dibenzthiazyldisulfid |
| 71,6 Gew.-Teile | Benzthiazyl-tert.-butyl-sulfenamid |
| 0,7 Gew.-Teile | Kupfer-(I)-benzthiazyl-2-mercaptid |
| 310 Gew.-Teile | tert.-Butylamin |

Die resultierende Suspension wurde auf 60°C aufgeheizt und mit 11 bar O₂ beaufschlagt. Ein Verbrauch an Sauerstoff setzte sofort ein. Sobald der Druck auf 10 bar abgefallen war, wurde erneut Sauerstoff zudosiert, so daß der Druck im Bereich von 10 bis 11 bar sich einpendelte. Nach etwa 2 Stunden war keine merkliche O₂-Aufnahme mehr zu registrieren und der Versuch wurde abgebrochen.

Man gab zu der Reaktionsmischung 400 Gew.-Teile H₂O und destillierte das Amin ab. Dann säuerte man mit 20 %iger Schwefelsäure schwach an (pH 3-4). Anschließend filtrierte man das Produkt ab, wusch den Filterkuchen neutral und trocknete das Produkt im Trockenschrank.
- Auswaage:: 87 Gew.-Teile (72 %)
- Schmelzpunkt:: 143°C
- Reinheit:: 99 %

### Beispiel 2

In einem Stahlautoklaven wurden vorgelegt

| | |
|---|---|
| 100 Gew.-Teile | 2-Mercaptobenzthiazol |
| 0,5 Gew.-Teile | Kupfer-(I)-benzthiazyl-2-mercaptid |
| 300 Gew.-Teile | tert.-Butylamin |

Die resultierende Suspension heizte man auf 60°C. Dann drückte man 11 bar Sauerstoff zu. Zunächst wurde nur langsam O₂ aufgenommen. Nach ca. 2 Stunden beschleunigt sich der O₂-Verbrauch rapide, bis schließlich (nach etwa 4,5 Stunden) keine Aufnahme von Sauerstoff mehr registriert wurde.

Das Produkt wurde abfiltriert und getrocknet. Das getrocknete Rohprodukt wurde in einer Soxhlet-Apparatur kontinuierlich mit Methyl-tert.-butyl-ether extrahiert. Nach etwa 5 Stunden wurde die resultierende Suspension filtriert, der Filterkuchen mit wenig Ether gewaschen und getrocknet.
- Auswaage:: 79 Gew.-Teile (65 %)
- Gehalt:: 99 %

## Patentansprüche

1. Verfahren zur Herstellung von Bis-benzthiazolylalkyl-sulfenimiden, dadurch gekennzeichnet, daß man Alkylamine mit gegebenenfalls substituierten 2-Mercaptobenzthiazolen in Gegenwart von Sauerstoff oder Sauerstoff-enthaltenden Gasen und Kupfer und/oder Kupferverbindungen in Anwesenheit von inerten, organischen Lösungsmitteln umsetzt.

2. Verfahren nach Anspruch 1, dadurch gekennzeichnet, daß man die Umsetzung bei Temperaturen von 20 bis 100°C und Drücken von 2 bis 100 bar durchführt.

3. Verfahren nach Ansprüchen 1 und 2, dadurch gekennzeichnet, daß man die Umsetzung in Gegenwart von 0,001 bis 1 Gew.-% Cu-Katalysator, bezogen auf die eingesetzte Menge an Mercaptobenzthiazol, durchführt.

4. Verfahren nach Ansprüchen 1 bis 3, dadurch gekennzeichnet, daß man als Katalysator Kupfer-(I)-benzthiazyl-2-mercaptid einsetzt.

5. Verfahren nach Ansprüchen 1 bis 4, dadurch gekennzeichnet, daß man als Aminkomponenten aliphatische Amine ohne Proton am α-Kohlenstoff einsetzt.

6. Verfahren nach Ansprüchen 1 bis 5, dadurch gekennzeichnet, daß man die Alkylamine als Lösungsmittel verwendet.

7. Verfahren nach Ansprüchen 1 bis 6, dadurch gekennzeichnet, daß man tert.-Butylamin einsetzt.
